# EUROPEAN PATENT APPLICATION

(11) **EP 1 462 083 A1**
(43) Date of publication of application: **29.09.2004**
(21) Application number: 02802710.0
(22) Date of filing: 01.11.2002
(51) Int. Cl.: A61K 7/00, A61K 7/06, A61K 7/075, A61K 7/08

(54) **SKIN LOTION COMPRISING AQUEOUS DISPERSION OF ULTRA-FINE TITANIUM PARTICLES**

(30) Priority: 06.11.2001 JP 2001340215
(71) Applicant: Phild Co., Ltd., Kyoto city, Kyoto pref. 604-8152 (JP)
(72) Inventor: HIRATA, Y., Phild Co, Ltd., 110, Goshohachiman-cho, Kyoto-shi, Kyoto 602-0023 (JP); UEDA, Y., Phild Co., Ltd., 110, Goshohachima-cho, Kyoto-shi, Kyoto 602-0023 (JP); TAKASE, H., Phild Co., Ltd., 110, Goshohachima-cho, Kyoto-shi, Kyoto 602-0023 (JP); SUZUKI, K., Phild Co., Ltd., 110, Goshohachima-cho, Kyoto-shi, Kyoto 602-0023 (JP)
(74) Representative: VOSSIUS & PARTNER
(86) International application number: PCT/JP2002/011421
(87) International publication number: WO 2003/039495

(57) **Abstract**

Use in cosmetic lotions and other applications a dispersion solution of super-fine titanium particles obtained by burning a mixture gas of oxygen and hydrogen in high-pressure water and using the combustion gas to melt titanium metal and thus causing super-fine titanium particles to micro-disperse in water, wherein such production process is implemented using an apparatus for producing a cosmetic lotion in which super-fine titanium particles are micro-dispersed that comprises a pressure-resistant container equipped with a high-pressure water tank, an injection nozzle for mixture gas of oxygen and hydrogen, a titanium metal bar, an ignition device and a combustion chamber.

## Description

### Field of the Invention

This invention relates to a cosmetic lotion made of high-function micro-dispersion water in which super-fine titanium particles are dispersed, a method and apparatus for producing the same, as well as a cosmetic material containing such micro-dispersion water of super-fine titanium particles as its water content.

### Background of the Invention

Titanium is a relatively new metal material discovered much more recently than iron, copper, aluminum, etc. The lightweight metal offering excellent strength at high temperature is already used in wide industrial applications, such as jet engine material for aircraft and spaceship, tube and tube plate comprising heat-exchangers used for nuclear or thermal power generation, and articles of daily use such as eyeglass frame and golf club head. The applications of titanium are expected to grow further.

A relatively large number of examples have been known to date regarding the use of titanium metal in articles of daily use, health/medical products and cosmetics. These examples include barber's scissors with titanium film coatings (Japanese Patent Application Laid-open No. 62-268584), the utilization of far-infrared ray through molten titanium metal (Japanese Patent Application Laid-open Nos. 61-59147, 1-155803 and 3-112849), bedding (Japanese Patent Application Laid-open No. 8-322695), cooking utensil (Japanese Patent Application Laid-open No. 9-140593), eye mask (Japanese Patent Application Laid-open No. 10-71168), health-maintaining equipment (Japanese Patent Application Laid-open Nos. 11-285541 and 11-285543), health band (Registered Japanese Utility Model No. 3045835) and health slippers (Registered Japanese Utility Model No. 3061466).

More than a few applications of titanium compounds for hair treatment purposes have been proposed (the examples in this field include Japanese Patent Application Laid-open Nos. 57-18606, 1-113313, 1-224310, 4-124121 and 5-170635, Japanese Patent Application Nos. 4-511289 and 5-504989, Japanese Patent Application Laid-open Nos. 11-315006 and 60-67210, Japanese Patent Publication No. 5-45565, Japanese Patent No. 1850423, Japanese Patent Application Laid-open No. 63-270620, Japanese Patent No. 2567861, Japanese Patent Application Laid-open No. 1-254617, Japanese Patent No. 2855205, Japanese Patent Application Laid-open No. 3-141216, Japanese Patent No. 2837196, Japanese Patent Application Laid-open Nos. 5-43431, 10-291927, 11-35434, 11-49650, 11-139946 and 11-292744). In addition, several cosmetic materials using titanium are also proposed (Japanese Patent Application Laid-open Nos. 58-72512, 60-87210, 62-45511, 1-294615 and 3-145413).

However, the examples of cosmetic materials using titanium as explained in the above published technologies involve addition of titanium in oxide or other form as pigment, and no technology has been known that utilizes high-function water containing titanium metal or apply titanium in similar forms. The example of producing water that contains titanium is limited to one that uses titanium metal in the cathode to produce drinking water through electroosmosis (Japanese Patent Application Laid-open No. 50-40779).

### Disclosure of the Invention

Use of titanium in bioactive materials, food materials, medical products, etc., is generating keen interests. However, the technologies available to date are far away from levels that enable practical applications and the actual bioactivity of titanium has not been uncovered.

The present invention aims to apply these almost infinite utilities of titanium in bioactive materials and health products in particular.

Specifically, the purpose of the present invention is to provide a cosmetic lotion comprising high-function water in which super-fine titanium particles are dispersed, wherein such super-fine titanium particles are produced by melting titanium metal in high-pressure water using a combustion gas of hydrogen and oxygen and then causing the obtained molten titanium to collide with high-pressure water, or to provide a cosmetic material that contains such high-function water as the base ingredient that acts upon the skin. The present invention also aims to provide a method and apparatus for producing the same.

In the context of the present invention, "titanium micro-dispersion water" refers to bioactive high-function water in which super-fine titanium particles are micro-dispersed and a very small amount of molten titanium is dissolved.

The basic feature of the present invention lies in the formation of a cosmetic lotion comprising high-function water in which super-fine titanium particles are micro-dispersed by way of melting titanium metal in high-pressure water using heat produced by burning a mixture gas of oxygen and hydrogen, and also of a cosmetic material containing such high-function water as its water content. Specifically, the present invention comprises (1) through (6) below:
(1) A cosmetic lotion comprising titanium micro-dispersion water in which super-fine titanium particles are micro-dispersed.
(2) A method for producing a cosmetic lotion in which super-fine titanium particles are dispersed, wherein a mixture gas of hydrogen and oxygen is burned in high-pressure water and the obtained combustion gas is used to melt titanium metal, thereby allowing super-fine titanium particles to disperse in water.
(3) An apparatus for producing a cosmetic lotion in which super-fine titanium particles are dispersed, comprising a pressure-resistant container equipped with a high-pressure water tank, an injection nozzle for mixture gas of oxygen and hydrogen, a titanium metal bar, an ignition device and a combustion chamber.
(4) The apparatus for producing a cosmetic lotion in which super-fine titanium particles are dispersed, as described in Claim 3, wherein a water electrolyzer for producing a mixture gas of oxygen and hydrogen is attached as an adjunct.
(5) A cosmetic material containing, as its water content, titanium micro-dispersion water in which super-fine titanium particles are dispersed.
(6) The cosmetic material described in (5) above, which is a creamy soap, cosmetic lotion containing medicament, milky lotion, cream, shampoo, rinse, hair tonic or hair cream.

Here, a cosmetic lotion obtained by the present invention, made of titanium micro-dispersion water in which super-fine titanium particles are dispersed, is specifically a skin lotion or hair growth stimulant/hair tonic that contains the aforementioned titanium micro-dispersion water as the main ingredient and also has coloring and aromatic agents added as necessary. A cosmetic material containing, as its water content, titanium micro-dispersion water in which super-fine titanium particles are dispersed uses titanium micro-dispersion water in which super-fine titanium particles are micro-dispersed, instead of normally used demineralized water, as the dispersant for a medicament that acts upon the skin or hair. It is provided as an aqueous solution, milky lotion or cream, and specific examples of such cosmetic material include a cosmetic lotion, milky lotion, cream, shampoo, rinse, hair tonic or hair cream containing creamy soap or medicament.

Generally in an aqueous dispersion solution or mixed solution in which super-fine particles of titanium or titanium oxide are simply dispersed, the titanium settles and separates within a short period of time. The most significant feature of the titanium micro-dispersion water obtained by the present invention is that the super-fine titanium particles remain micro-dispersed for a long period of time without settling or separating. The titanium melted by combustion heat disperses in high-pressure water and remains micro-dispersed in a stable condition without settling or otherwise separating from water.

The titanium micro-dispersion water obtained by the present invention therefore exhibits the surprising, remarkable effect of providing a bioactive cosmetic lotion through an interaction of water molecules and super-fine titanium particles.

In general, healthy skin retains 10 to 20% of water in its keratin layer to maintain the skin's suppleness. If the water content in the keratin layer drops to 10% or less, the skin will become dry, and dullness, rough skin, generation of white powder comprising separated dead skin cells, or chapped skin will result. To treat such skin, water and oil must be replenished externally to restore a normal moisture/oil balance in the skin. On the other hand, hair is damaged by external irritations such as friction, heat from the sun, perm chemicals and inappropriate cuts, as well as by malnutrition caused by an unbalanced diet or drastic weight loss. Since damaged hair has no self-repairing ability, it must be protected externally to prevent further damage.

Cosmetic lotions and treatment agents are used for the aforementioned purposes. Water and oil are two important constituents of the skin and hair. In order to maintain supple skin, the skin's keratin layer retains the water secreted from sweat glands or absorbed through the skin, while evaporation of the retained water is prevented by the sebum cutaneum. Water is also absorbed into the hair cortex, which is a component of hair, and adds moisture and a supple look to the hair while making the hair more flexible so as to let it bounce as the body moves. Oil forms an oil film over the hair cuticle and adds shine and gloss to the hair while minimizing friction and preventing damage to the hair cuticle.

The high-function titanium micro-dispersion water obtained by the present invention has been confirmed to provide excellent skin activation effects such as repairing small cuts in the skin caused by razor blades, etc., as well as remarkable repair effects on damaged hairs such as those having invisible nicks on the surface, which are not available with the cosmetic lotions or treatment agents currently available in the market.

In addition to the aforementioned cosmetic lotion or cosmetic material, the high-function titanium micro-dispersion water obtained by the present invention can also be utilized in health products such as motor-function enhancing creams, medical products such as antibacterial agents, UV-protection products, healthy drinking water, health supplements, food preservatives, freshness-keeping agents for food, insect repellents, deodorants, and so on.

The mechanism as to why such titanium micro-dispersion water in which super-fine titanium particles are dispersed provides excellent bioactive functions is not yet clear. The inventors are working diligently to find scientific explanations for these functions.

### Brief Description of the Drawings

Fig. 1: Production flow chart of a cosmetic lotion made of titanium micro-dispersion water as proposed by the present invention
Fig. 2: Apparatus for producing a cosmetic lotion made of titanium micro-dispersion water as proposed by the present invention
Fig. 3: Table showing the analysis test results of titanium micro-dispersion water as proposed by the present invention

### Description of the Symbols

1: Production apparatus for titanium micro-dispersion water as proposed by the present invention
2: Pressure-resistant container for producing titanium micro-dispersion water
3: Electrolyzer/material gas generator
5: High-pressure water tank
6: Combustion chamber
7: Pressure regulating valve
8: Outlet for titanium micro-dispersion water
9: High-pressure water
10: Titanium metal bar
11: Ignition device
12: Molten titanium metal
13: Feed cylinder
14: Mixture gas injection nozzle
16: Hydrogen feed channel
17: Oxygen feed channel
18: Cathode
18': Anode
19: Partition
20: Water

### Best Mode for Carrying Out the Invention

A cosmetic lotion made of titanium micro-dispersion water or cosmetic material containing titanium micro-dispersion water as its water content, as obtained by the present invention, is a new product not heretofore available.

The present invention also provides a new method and apparatus for producing a cosmetic lotion made of water that contains titanium as super-fine particles, wherein the production of such titanium micro-dispersion water is based on melting titanium metal not through general methods that are commonly used to melt metal, but by using heat generated from burning oxygen and hydrogen and causing the obtained molten titanium to disperse in high-pressure water.

Specifically, after studying ways to efficiently and economically produce a cosmetic lotion made of titanium micro-dispersion water, as well as ways to apply such micro-dispersion water to bioactivation purposes, the inventors conceptualized a method to burn hydrogen and oxygen, insert a bar made of pure titanium metal into the combustion atmosphere to heat the titanium bar, and cause the obtained molten titanium to collide with high-pressure water and disperse in water as super-fine titanium particles, wherein such combustion of hydrogen and oxygen is caused in water so as not to produce substances other than water and titanium metal.

The method proposed by the present invention requires that the amounts of hydrogen and oxygen to be burned, reaction pressure and feed rate of titanium metal be controlled, in order to add excellent bioactivity to the obtained product. In the aforementioned production method, an appropriate filter system is also necessary because not only super-fine titanium particles but also a trace amount of fine titanium-oxide particles will be produced in water.

A method for producing a cosmetic lotion made of titanium micro-dispersion water as obtained by the present invention in high-pressure water through the aforementioned method, as well as an apparatus to embody this method, are explained according to the drawings.

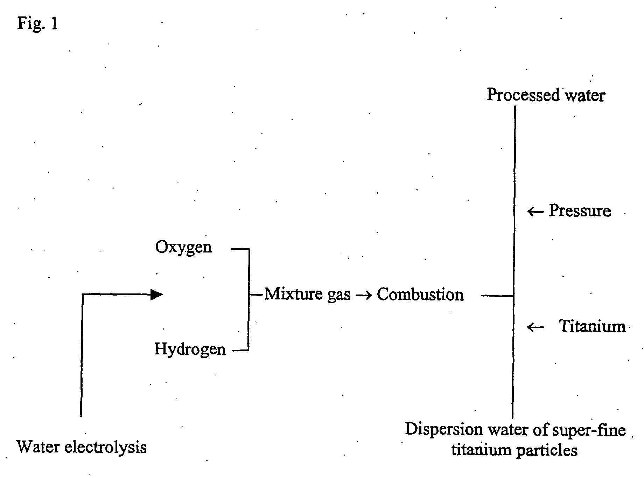

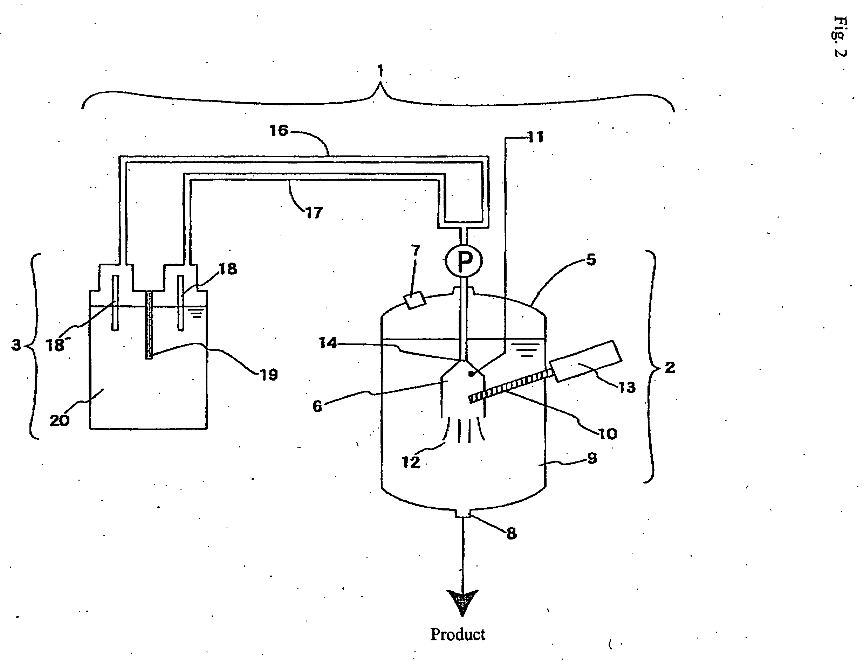

Fig. 1 is a production flow chart of a cosmetic lotion made of titanium micro-dispersion water as proposed by the present invention, while Fig. 2 shows an apparatus for producing a cosmetic lotion made of titanium micro-dispersion water as proposed by the present invention.

The production apparatus for titanium micro-dispersion water as proposed by the present invention (1), as shown in Fig. 2, comprising a pressure-resistant container (2) for producing high-pressure water in which molten titanium is dispersed, an electrolyzer/material gas generator (3) and a filter system to filter titanium micro-dispersion water (not illustrated).

The basic structure of the pressure-resistant container (2) proposed by the present invention comprises a production apparatus for titanium micro-dispersion water in which super-fine titanium particles are dispersed, wherein such apparatus comprises a high-pressure water tank (5), an injection nozzle for mixture gas of oxygen and hydrogen (14), a combustion chamber (6) and a titanium metal bar (10). A water electrolyzer (3) for supplying material hydrogen and oxygen and a filter system that filters produced titanium micro-dispersion water are attached as adjuncts.

The pressure-resistant tank (2) proposed by the present invention comprises the high-pressure water tank (5) made of metal, or preferably steel. In this high-pressure water tank (5), a mixture gas of the hydrogen and oxygen generated by the electrolyzer (3) and supplied through a hydrogen feed channel (16) and oxygen feed channel (17) is injected at high pressure from an injection nozzle (14) into the combustion chamber (6). The titanium metal bar (10) is fed by a feed cylinder (13) into the combustion chamber (6) successively in accordance with the melting amount of titanium. The mixture gas of hydrogen and oxygen is ignited by an ignition device (11), and molten titanium metal is released into high-pressure water (9). The molten titanium becomes super-fine particles in the high-pressure water, and the high-pressure water (9) containing these super-fine titanium particles is retrieved from the high-pressure water tank via an outlet (8) at the bottom of the tank and then filtered by an appropriate filter system.

As for the material gas generator (3), hydrogen and oxygen may be fed directly to the high-pressure water tank (5) through respective high-pressure cylinders. However, the hydrogen and oxygen produced by water electrolysis as proposed by the present invention are pure gases, and therefore use of an electrolyzer has the advantage of enabling an efficient supply of combustion gas of oxygen and hydrogen mixed at a theoretical ratio of 1 to 2 and thus preventing impurities other than titanium from mixing into the micro-dispersion.

The present invention provides an example of generating hydrogen and oxygen via electrolysis of water (20) in the material gas generator (3), as material gases used in the production of titanium micro-dispersion water. A cathode and an anode are denoted as (18) and (18'), respectively.

This apparatus injects hydrogen and oxygen, generated via electrolysis and supplied through the hydrogen feed channel (16) and oxygen feed channel (17), into the combustion chamber (6) from the nozzle (14) using a pump, and causes the mixture gas to burn completely to achieve a state of perfect steam gas of ultrahigh temperature. The pure titanium metal bar (10) is inserted into this combustion gas to be heated and melted. The titanium metal bar is inserted via the cylinder (13) at a constant rate according to the melting amount. To melt the titanium bar, the mixture ratio of hydrogen and oxygen must be strictly controlled at 2 to 1. Also, a pressure regulating valve (7) must be provided to regulate the pressure inside the high-pressure water tank.

The molten titanium (12), heated to high temperature and thus melted in the combustion chamber (6), is released from the combustion chamber (6) into high-pressure water (9), where the titanium collides with the high-pressure water to become super-fine particles. It is considered that a part of titanium takes on a crystalline structure in this process.

The produced super-fine titanium particles in water have very strong hydrophobicity and therefore remain micro-dispersed in water in a stable condition without settling even when a coagulating agent is added.

As for the operation of this apparatus, high-pressure hydrogen and oxygen are injected into the high-pressure water tank (5) from the nozzle (14) using a pump, the gas is ignited by the injection device (11) to achieve a state of perfect steam gas of ultrahigh temperature, and the pure titanium metal bar (10) is successively inserted into the combustion gas and melted.

In this apparatus, hydrogen and oxygen must be burned in water so as not to produce substances other than water and super-fine titanium particles. Combustion must occur under high pressure in order to burn hydrogen and oxygen in water in impurity-free state. In addition, the position where the titanium metal bar is inserted must be where the mixture gas burns completely to achieve a state of perfect steam gas of ultrahigh temperature.

Other characteristics of the present invention include the use of thus produced titanium micro-dispersion water, in which super-fine titanium particles are micro-dispersed, as a material for cosmetic lotions and other cosmetic products as well as health products, food materials, drugs and quasi-drugs after necessary refining. The produced titanium micro-dispersion water contains a small amount of fine titanium-oxide particles generated via bonding of titanium and oxygen and must therefore be filtered and refined as necessary.

So as not to remove the produced super-fine titanium particles more than necessary, filtering should preferably be implemented not by using ion exchange or reverse-osmosis membrane, but by using the filter system as explained below that can yield titanium micro-dispersion water suitable for a desired purpose. For example, filtering the high-pressure water discharged from the high-pressure water tank sequentially through hollow-fiber membranes is beneficial in terms of enhancing the characteristics of titanium micro-dispersion water and extending the filter life, and such filter system will provide titanium micro-dispersion water that can meet the required standards governing food and sanitation, cosmetics and drugs.

The constituent analysis of titanium micro-dispersion water as obtained by the present invention found a specified amount of super-fine titanium particles and a very small amount of titanium in the high-pressure water.

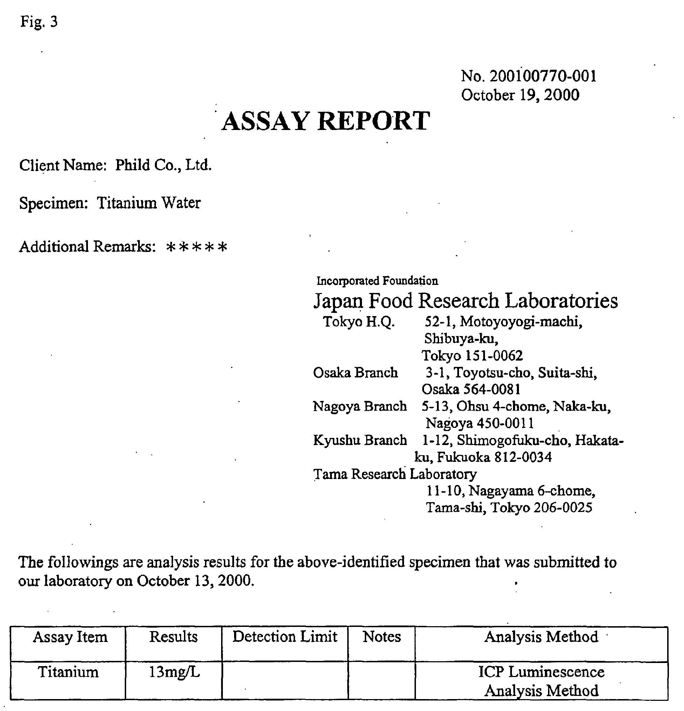

The analysis results of titanium micro-dispersion water as obtained by the present invention are shown in Fig. 3.

### Example:

An embodiment of the present invention is explained in details using an example. Note, however, that the present invention is not limited to this example.

Fig. 2 shows an example of the representative apparatus proposed by the present invention, wherein the apparatus comprises a high-pressure water tank (5), an injection nozzle for mixture gas of oxygen and hydrogen (14) and a titanium metal bar (10) and is used for producing a cosmetic lotion made of titanium micro-dispersion water in which super-fine titanium particles, generated as a result of collision between molten titanium and high-pressure water, are dispersed.

The high-pressure water tank (5) is a metal pressure-resistant tank providing resistance under ultrahigh pressures, wherein a mixture gas of hydrogen and oxygen supplied through the hydrogen feed channel (16) and oxygen feed channel (17) is injected into a combustion chamber (6) through the injection nozzle (14), and the titanium metal bar (10) is fed into the combustion chamber via a cylinder (13). The pressure inside the high-pressure water tank (5) must be controlled using a pressure regulating valve (7). The mixture gas is ignited by an ignition device (11), and the obtained molten titanium (12) is released into, and collides with, high-pressure water to become super-fine titanium particles. Thus produced titanium micro-dispersion water in which super-fine titanium particles are dispersed is retrieved from the tank via an outlet (8).

As for the operation of this apparatus, as mentioned above hydrogen and oxygen are fed under high pressure into the high-pressure water tank (5). The mixture gas is injected from the nozzle (14) and ignited by the ignition device (11) to be burned completely to achieve a state of perfect steam gas of ultrahigh temperature. The titanium metal bar (10) is inserted into this combustion gas and melted. Titanium (12), thus heated to high temperature and melted in the nozzle, is released into, and collides with, high-pressure water to become super-fine particles. At this time, a part of titanium takes on a crystalline structure, to which titanium atoms will rearrange themselves to form particles of near-spherical shape, thus attaining an energetically stable state.

In the aforementioned production method, in order to produce one ton of titanium micro-dispersion water a mixture gas of around 3.5 atmospheres should be injected at approximately 4 to 6 liters per second into the high-pressure water tank containing water pressured to around 1.5 to 2.5 atmospheres, while the titanium metal bar should be fed at 0.5 kg over two hours. An excessive gas pressure may damage the structural components of the apparatus, while too low a pressure will cause water to enter the combustion chamber and allow the heated/melted titanium to dissipate into air in air bubbles, thus making less ideal the production condition of titanium micro-dispersion water containing super-fine titanium particles.

The produced titanium micro-dispersion water contains titanium that remains in micro-dispersed state without using any active agent. This water is retrieved from the outlet and fed to a filter system as appropriate. The filter system comprises filter membranes of 50 microns, 25 microns, 3 microns, 0.5 micron and 0.1 micron, and by passing the produced water through these filter membranes sequentially a final titanium micro-dispersion water containing a specified amount of super-fine titanium particles and a very small amount of dissolved titanium is obtained.

### Trial Test of Skin Lotion Made of Obtained Titanium Micro-Dispersion Water

Ten adult female subjects were instructed to use titanium micro-dispersion water in which super-fine titanium particles are dispersed, and the effects and efficacies of the treatment were verified.

The subjects participating in the test applied to their face a cosmetic lotion made of the titanium micro-dispersion water obtained under the aforementioned condition.

### Use Conditions and Test Results

1. 10 female subjects (The subjects used the water at varying frequencies)
2. Efficacies

| | | | |
|---|---|---|---|
| Dull skin complexion improved | 6 persons | Wrinkles disappeared | 2 persons |
| Skin became suppler | 3 persons | Wrinkles became less | 3 persons |
| Skin became smoother | 5 persons | | |

### Trial Test of Hair Growth Stimulant/Hair Tonic Made of Obtained Titanium Micro-Dispersion Water

The effectiveness, as a hair growth stimulant/hair tonic, of a cosmetic material made of the titanium micro-dispersion water obtained in the same manner was tested.

Ten adult male and female subjects were instructed to apply to their hair titanium micro-dispersion water in which super-fine titanium particles are dispersed, and the effects and efficacies of the treatment were verified.

The subjects participating in the test used a hair growth stimulant/hair tonic made of the titanium micro-dispersion water obtained under the aforementioned condition by washing their hair with the water or spraying/applying the water to their hair.

### Use Conditions and Test Results

Use conditions: Washing hair in a basin (3 liters per wash)
: Spraying onto hair with a spray (10 cc per application)
: Applying onto hair using a brush (10 cc per application)
1. 10 male and female subjects (The subjects used the water at varying frequencies)
2. Efficacies

| | |
|---|---|
| Hair became less tangling | 5 persons |
| Fallen hair became less | 8 persons |
| Hair became shiner | 7 persons |
| Hair became darker | 1 person |
| Hair became denser | 6 persons |
| Scalp became smoother | 7 persons |
| Dandruff became less | 7 persons |

As evident from the above test results, many of those who used the titanium micro-dispersion water obtained by the present invention cited improved skin conditions such as suppler and smoother skin. Also, many of those who washed their hair with the titanium micro-dispersion water or applied/sprayed it to their hair cited reduction in fallen hair, increase in hair density, recovery of damaged hair, improved hair condition, smoother scalp, reduced dandruff and other improvements. Therefore, it is found that a cosmetic lotion made of titanium micro-dispersion water as obtained by the present invention would be effective in improving the skin condition and promoting hair growth. The skin activation effects of such water will likely help reduce and prevent skin pigmentations and also prevent aging of the skin, thereby delivering remarkable effects as drugs, cosmetics, and so on.

Based on the confirmed effects of titanium micro-dispersion water as explained above, use of titanium micro-dispersion water obtained by the present invention as the water content for various cosmetic materials, instead of normal demineralized water, will likely produce high-function cosmetic materials offering the efficacies of active ingredients contained in the cosmetic material as well as the effects of titanium micro-dispersion water.

### Industrial Field of Application

The present invention, whose structure is explained above, provides a cosmetic lotion comprising newly developed titanium micro-dispersion water, as well as its production method and apparatus, and allows for efficient production of a cosmetic material whose main ingredient is bioactive titanium micro-dispersion water containing super-fine titanium particles in dispersed state. Trial tests also found that cosmetic materials comprising thus obtained titanium micro-dispersion water are also useful as material for various cosmetic products, healthy drinking water, health supplements, food preservatives, freshness-keeping agents for food, insect repellents and deodorants, which suggests beneficial effects of such water in industrial applications.

## Claims

1. A cosmetic lotion comprising titanium micro-dispersion water in which super-fine titanium particles are dispersed.

2. A method for producing a cosmetic lotion in which super-fine titanium particles are dispersed, comprising:
burning a mixture gas of hydrogen and oxygen in high-pressure water; and
melting titanium metal using the obtained combustion gas, thereby allowing super-fine titanium particles to disperse in water.

3. An apparatus for producing a cosmetic lotion in which super-fine titanium particles are micro-dispersed, comprising a pressure-resistant container equipped with a high-pressure water tank, an injection nozzle for mixture gas of oxygen and hydrogen, a titanium metal bar; an ignition device and a combustion chamber.

4. The apparatus for producing a cosmetic lotion in which super-fine titanium particles are micro-dispersed, as described in Claim 3, further comprising a water electrolyzer for producing a mixture gas of oxygen and hydrogen.

5. A cosmetic material containing, as its water content, titanium micro-dispersion water in which super-fine titanium particles are micro-dispersed.

6. The cosmetic material as described in Claim 5, which is a creamy soap, cosmetic lotion containing medicament, milky lotion, cream, shampoo, rinse, hair tonic or hair cream.
